(19) **Europäisches Patentamt** / **European Patent Office** / **Office européen des brevets**

(11) **EP 4 745 234 A1**

(12) **EUROPEAN PATENT APPLICATION**
published in accordance with Art. 153(4) EPC

(43) Date of publication:
**20.05.2026  Bulletin 2026/21**

(21) Application number: **24838947.0**

(22) Date of filing: **15.07.2024**

(51) International Patent Classification (IPC):
*C12N 13/00* (2006.01)  *C12M 1/42* (2006.01)

(86) International application number:
**PCT/ES2024/070450**

(87) International publication number:
**WO 2025/012507 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:  **13.07.2023  ES 202330589**

(71) Applicant: **Consejo Superior de Investigaciones Científicas (CSIC)
28006 Madrid (ES)**

(72) Inventors:
- **GONZÁLEZ GÓMEZ, Icíar**
  28006 Madrid (ES)
- **SALEHI JIMÉNEZ, Nadia**
  28006 Madrid (ES)
- **VALDIVIESO MARTÍNEZ, Alba**
  28006 Madrid (ES)
- **LUZURIAGA GONZÁLEZ, Jon**
  28006 Madrid (ES)

(74) Representative: **Pons IP
Glorieta Rubén Darío 4
28010 Madrid (ES)**

(54) **ULTRASONIC DEVICE AND METHOD FOR GENERATING THREE-DIMENSIONAL CELL SPHEROIDS**

(57)     The invention relates to an ultrasonic device and an associated method for generating three-dimensional cell spheroids. The device comprises a polymer structure (2) of acoustically soft material with acoustic impedance similar to that of a liquid medium to be treated, which in turn comprises a set of containers (3) coupled to an ultrasonic actuator (1) on the bottom part thereof. The device allows three-dimensional cell spheroids to be generated from liquid suspensions in short periods of time of a few minutes and is also valid for particles or other micro-elements, with applications in research or analysis in tumour biology, tissue engineering and clinical analysis and research concerning molecular targets in personalised medicine, thus replacing the use of animals.

**FIG. 2**

**Description**

**OBJECT OF THE INVENTION**

The present invention belongs to the field of acoustofluidics, combining aspects of millifluidics and acoustophoresis.

Specifically, the invention relates to an ultrasonic device and an associated process for generating three-dimensional cell spheroids from liquid suspensions in short periods of time of a few minutes and is also valid for particles or other micro-elements.

**BACKGROUND OF THE INVENTION**

Currently, the use of three-dimensional (3D) in-vitro models represents an effective bridge to animal models. These 3D models mimic characteristics of the in-vivo environment and are presented as a tool for the study of cell, tissue organ and tumour behaviour. They represent a key tool for transdisciplinary research in cancer biology, tissue engineering, and the search for optimised drugs for personalised medicine.

The advantages of these three-dimensional in-vitro models, and particularly the tumour spheroids or 3D models are:

-    cell morphology and signalling are often more physiological than routine 2D cell culture,
-    they allow for rapid experimental manipulations and hypothesis testing, and
-    they allow better quality real-time and/or still images by microscopy than in animals.

Many 3D models have been developed from isolated cells, such as cell lines, dissociated tissues or stem cells. A widely used strategy is the implantation of culture cells into 3D scaffold structures as tissue-like aggregates.

Tumour spheroids consist of 3D aggregates of cells from monocultures or co-cultures. Spheroids can be formed from hanging drops, in bioreactors, or in 3D matrices, and can be created either from cells in a population or co-culture, or from multi-cellular mixtures of tumour, stromal and immune system cells.

It is widely known that these aggregates can mimic tumour behaviour more effectively than 2D cultures because the spheroids contain well-oxygenated, hypoxic cells that secrete tumour-associated cytokines. These spheroids may prove valuable for the development of efficient drugs by replacing the use of animals in tests.

Spheroids can also be used to study the adherent properties of tumour cells, and allow the analysis of their individual and collective behaviours.

However, long laboratory periods of 14 hours or even several days are required to induce tumour spheroid formation by conventional systems.

It is also well known that epithelial cells, and in particular tumour cells exposed to ultrasound, acquire oscillatory motions when exposed to an acoustic wave and, at certain amplitudes below the threshold of cell damage, are exposed to a radiation force that drives them towards acoustic pressure nodes established under standing wave conditions, known as acoustophoresis.

Over the past few decades, multiple devices have been developed based on this mechanism for sorting and separation applications, but few for the formation of 3D cell spheroids.

Firstly, a device consisting of a silicon plate containing 10x10 containers, each measuring 0.3x0.3 mm$^2$, is known. This device establishes an acoustic pressure node in the centre of each container at a frequency close to 2.5 MHz, where it is established due to the rigidity of the walls, and which establishes a standing wave $\lambda$ for acoustic pressures between 0.5-1MPa.

However, this device has certain disadvantages associated with the high manufacturing cost of silicon microfluidic devices, which also require the use of clean rooms and restrictive conditions in the manufacturing process. As a result, mass production for clinical or biomedical use has never taken place. Furthermore, these devices are unfeasible for any application requiring disposability and do not conform to European Union sustainability requirements.

Another known device for 3D cell aggregation consists of a conventional culture plate exposed from below to standing waves at a frequency of 110 kHz and 70 V peak-to-peak voltages applied by a Langevin transducer. At such a low frequency, the wavelength is about 15 mm, which is two orders of magnitude longer than in the previous work. This generates radiation forces ten times lower than that produced by a wave applied at 1 MHz, leading to cell conduction times and collection at the pressure nodes that are also about one order of magnitude higher.

In light of the above, there is a need for a sustainable and low-cost device for the rapid formation of cell spheroids for research purposes in tumour biology, tissue engineering, clinical analysis and the search for molecular targets in personalised medicine, allowing the use of animals to be replaced and complying with European regulations on the sustainability of technological developments.

**DESCRIPTION OF THE INVENTION**

The present invention relates to an ultrasonic device and an associated process for generating three-dimensional cell spheroids from liquid suspensions in short periods of time of a few minutes and is also valid for particles or other micro-elements.

With regard to the applications of the present invention, it is applicable, for example, to research or analytical purposes in tumour biology, tissue engineering and clinical analysis and search for molecular targets in personalised medicine, allowing the use of animals to be replaced.

The device comprises a disposable polymer structure comprising a set (array) of containers or wells. The containers comprise a base and four sides, and are intended to contain a liquid medium to be treated, in which the spheroids are to be generated. The polymer structure has an acoustic impedance similar to that of the liquid medium to be treated. The acoustic impedance Z is the product of the density of the medium multiplied by the propagation speed of the wave through the same ($Z = \rho c$). The impedance in plastics is $Z_{plastics}$~2.1 - 2.4 MRayls and the impedance in a liquid suspension is $Z_{liquid\ susp.}$ ~1.5-1.7 MRayls.

Preferably, the base of each container of the polymer structure is a square cross section, with sides between 2 and 4 mm long, and the four sides are flat walls between 8 and 12 mm high.

In addition, the device comprises an ultrasonic (piezoelectric) actuator concentrically coupled below the containers, in contact with their base. The device further comprises a signal generator for providing sinusoidal electrical waves to the ultrasonic actuator.

The ultrasonic actuator is preferably a piezoelectric ceramic (piezoelectric transducer), which is resonant in thickness mode at a certain frequency, mechanically coupled under the bases of the containers. Once it receives the electrical signal from the signal generator at the desired frequency, it transforms it into mechanical vibrations which it transfers to the structure of the set of containers to which it is coupled.

The polymer structure with the set of containers may be made of a transparent material, and as indicated above, its acoustic impedance must be similar to that of the liquid medium to be treated, with a difference in values of less than 60% between the two media: liquid suspension and plastic material of the structure. An example of a polymer material that can be used is polyethylene.

The physical properties of the polymers used in the polymer structure are very different from those of the ultrasonic actuator, which is preferably a piezoelectric ceramic, the acoustic impedance of which is much higher than that of the plastic material $Z_{piezoelectric}$=17.3 MRayls, about seven times higher, so its acoustic response is very different and closer to that of the liquid medium. In addition, polymers are highly absorbent media, in which a plane wave is rapidly transformed into three-dimensional waves. In this way, instead of standing waves being established between the side walls of each of the containers, a 3D wave is generated in each of the containers produced by three-dimensional vibrations established in the plastic structure of the array or set of containers.

The properties of these polymeric materials make it very difficult to achieve standing wave conditions inside the containers, but they have the advantages of being easy to manufacture, low cost and easy to handle. In addition, there are several frequencies with optimal acoustic pressure distributions for the collection of particles or cells rather than a single frequency that imposes the resonance of the containers at a frequency determined by the dimensions thereof, which are those applied by the signal generator to the ultrasonic actuator.

The three-dimensional mechanical vibrations set up in the polymeric structure with the set of containers are frequency specific and at the interfaces of each container with the contained liquid medium are transferred to the container, generating a complex 3D distribution of acoustic pressures different for each container and frequency.

Once the wave is established in the liquid phase contained in each container, a nonlinear interaction occurs between the incident wave and the scattered wave created by each cell in the suspension, generating a hydrodynamic and time-independent radiation force acting thereon and driving it individually towards defined acoustic equilibrium zones inside each container, corresponding to pressure nodes.

The particles or cells dragged by the radiation force towards these positions collect in short periods of time less than five minutes. Once collected at the acoustic pressure nodes within the containers, the cells aggregate to form stable cell spheroids, in which the cells remain alive during subsequent culture processes.

Regarding the specific operation and method of the invention, firstly, the signal generator activates the actuation of the ultrasonic actuator, converting the electrical signal into mechanical vibration, with maximum amplitude at a resonance frequency of its thickness mode.

In order to identify the optimum actuation frequencies, an experimental electrical characterisation is carried out. This is due to the large number of parameters involved in these processes: dimensions and geometry of the polymer structure and containers, number and spatial distribution of the containers, concentration of the cells in the suspension, typology of the cells in suspension and location of the ultrasonic actuator with respect to the polymer structure.

In addition, the following requirements must be taken into account when identifying preferred resonance frequencies:

- formation and consolidation of the cell aggregate in the shortest possible time to optimise acoustic energy consumption,
- compaction of cell aggregates, and
- generation of cell aggregates in the maximum number of containers.

As the device uses the ultrasonic transducer, it is appropriate to search in the vicinity of the singular frequencies determined in the electrical conductance measurements and to directly check the formation of the cell aggregates by visual observation in the containers over time.

Therefore, sinusoidal electrical waves are applied to the containers of the polymer structure with the ultrasonic actuator and generated in the signal generator with a variable frequency between 450kHz and 520kHz. A measurement of the conductance of the ultrasonic actuator is then obtained for each of the applied frequencies and at least one operating frequency is determined for which the conductance is at a maximum.

Turning to the operation of the device, secondly, the standing wave at the working frequency set in the ultrasonic actuator is transmitted into the polymer structure, where complex three-dimensional vibration modes are established. The three-dimensional mechanical vibrations set in the polymer structure are frequency-specific and are transferred to the liquid sample through the sides (interfaces) of each container, generating a three-dimensional acoustic pressure distribution specific to each container and frequency, with spatial amplitude gradients within each container.

Once the wave is established in the liquid phase contained in each container, a nonlinear interaction occurs between the incident wave and the scattered wave created by each cell in the suspension, generating a time-independent radiation force $FR_p$ acting thereon and driving it individually towards defined acoustic equilibrium zones inside each container, corresponding to pressure nodes. This is a hydrodynamic force proportional to the volume and shape of each cell or particle in suspension, to the frequency of the applied acoustic wave, to the spatial distribution of the pressure in each container, as well as to the viscoelastic properties of the cells (or particles) and of the liquid medium in which they are immersed. The radiation force drives each cell or particle towards the nearest established acoustic equilibrium positions, and is calculated for a standing wave as:

$$FR_p = \frac{\pi P_0^2 V_p \beta_l}{2\lambda} \varphi(\rho_p, \beta_p, \rho_l, \beta_l) sin\left(\frac{4\pi x}{\lambda}\right)$$

where $\varphi(\rho_p, \beta_p, \rho_l, \beta_l) = \frac{5\rho_p - 2\rho_l}{2\rho_p + \rho_l} - \frac{\beta_p}{\beta_l}$ is defined as the acoustic contrast factor, $"\varphi"$, which defines the relationship between densities and adiabatic compressibilities of particles or cells ($\rho_p, \beta_p$) and the liquid medium in which they are immersed ($\rho_l, \beta_l$), respectively.

The distance to the nearest pressure node is defined by $"x"$ and $"\lambda"$ represents the length of the acoustic wave in the medium. For its part, $\varphi$ it indicates the direction of movement of the particles or cells, either towards the pressure nodes when it is positive ($\varphi > 0$) or towards the antinodes when it is negative ($\varphi < 0$), where the acoustic pressure has maximum amplitude.

The acoustic contrast factor is positive both for particles of the material of the container and for epithelial cells, which are driven towards the acoustic pressure nodes.

The conduction process from their initial positions within the container to the node requires different times and trajectories depending on the initial position of the cells (or distance to the pressure nodes), which means that this process is not instantaneous, but requires a time interval of up to several minutes depending on the density of cells in the suspension, as well as the amplitude of the applied acoustic wave.

The particles or cells dragged by the radiation force towards these positions collect in short periods of less than five minutes in the device of the invention. Thus, an aggregation process starts and remains stable after the ultrasonic action. The size of the cell aggregate increases over time as more cells arrive from different positions within the container, creating a nucleation of the cluster where the cells aggregate.

Cell aggregates can acquire irregular or spherical geometries, depending on the shape of the established acoustic pressure node.

## DESCRIPTION OF THE DRAWINGS

As a complement to the description provided and for the purpose of helping to make the features of the invention more readily understandable, in accordance with a practical preferred exemplary embodiment thereof, said description is accompanied by a set of drawings which, by way of illustration and not limitation, represent the following:

Figures 1 and 2 show a top view and a side cross section of a diagram of the device with the structure of nine containers arranged in a 3x3 array.

Figure 3 shows a graph of the relationship between the frequency of the input signal (f) and the measured conductance (G) of the ultrasonic actuator.

Figure 4 shows a photograph of the formation of various spheroids of PANC-1 pancreatic cancer cells, ultrasonically induced over a time of approximately 4 minutes.

## PREFERRED EMBODIMENT OF THE INVENTION

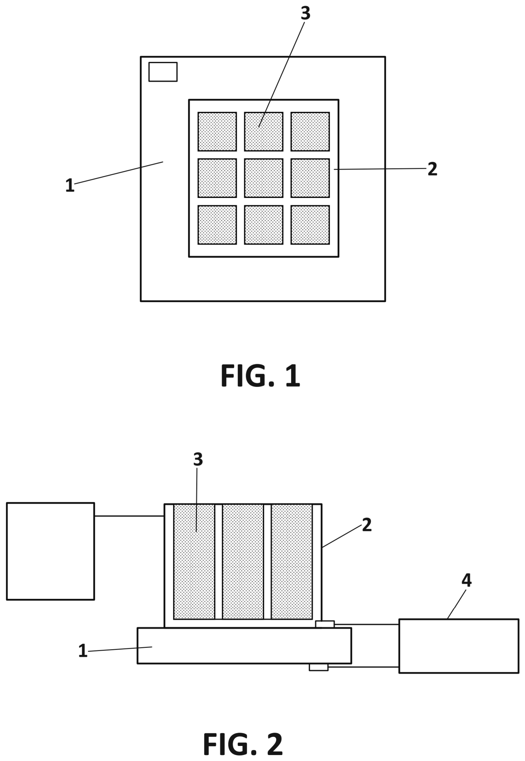

A preferred embodiment of the ultrasonic device and associated method for generating three-dimensional cell spheroids from liquid suspensions in short periods of time of a few minutes and is also valid for particles or other micro-elements, is described below with the help of Figures 1-4.

As shown in Figures 1 and 2, the device comprises an ultrasonic actuator (1), particularly a piezoelectric ceramic, mechanically coupled to a polymer structure (2) with millifluidic containers (3) in which a three-dimensional ultrasonic wave is set to act on each particle or cell in suspension, driving them towards acoustic equilibrium positions where they collect to form aggregates.

The containers (3) have a square geometry as does the ultrasonic actuator (1), which measures 30x30 $mm^2$ in area and has a thickness of 1.5mm, and is designed to resonate in thickness mode, preferably at a frequency of 491 kHz.

The ultrasonic actuator (1) and the polymer structure (2) are concentrically coupled. The device further comprises, as shown in Figures 1 and 2, a signal generator (4), connected to the ultrasonic actuator (1), to supply it with sinusoidal electrical waves.

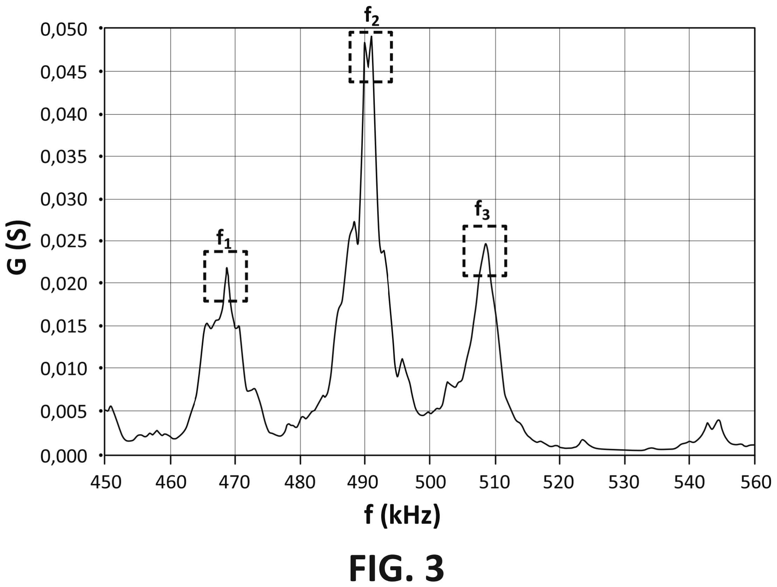

Figure 3 shows the relationship between the frequency of the input signal (f) generated by the ultrasonic actuator (1) and its conductance (G). The graph shows three peaks where the conductance is maximum, at $f_1$ = 469 kHz, $f_2$ = 491 kHz and $f_3$ = 508 kHz, which is where the device is in resonance and shows its maximum acoustic vibration capacity.

Of the three resonance frequencies of the device, the one found to be suitable for rapid cell spheroid formation was chosen: $f_2$ = 491 kHz. Low pressure levels of $P_{max}$ = 250 kPa have been required to induce cell aggregates and short ultrasonic irradiation times, below the threshold of cell damage.

Moreover, the polymer structure (2) can have different configurations: with a single container (3), with four containers (3) arranged in a 2x2 two-dimensional array or with nine containers (3) arranged in a 3x3 two-dimensional array, all of them respectively centred on the ultrasonic actuator (1).

Specifically, in the embodiment with nine containers (3), the polymer structure (2) has dimensions $127.8x85.5x15.0 \pm 0.2$ $mm^3$ and the containers are geometrically distributed in 3x3 containers (3).

In the embodiment with four containers (3), the polymer structure (2) has dimensions $11x11x0.2 \pm 0.2$ $mm^3$ and the four containers (3) are geometrically distributed in 2x2 containers (3).

Finally, in the embodiment with a single container (1), the polymer structure (2) has dimensions $5.5x5.5x0.2 \pm 0.2$ $mm^3$.

The dimensions of each container (3) are $3.4x3.4x6.4$ $mm^3$, with a volume of 74 $\mu$l. Liquid samples of 50 $\mu$l are introduced into each of the containers (3). The distance between adjacent containers (3) is 4.5 mm.

Lastly, the polymer structure (2) and the containers (3) are preferably made of polyethylene with a density $\rho$ = 1.01 g/cm. Furthermore, in one aspect of the invention, a transparent polymer is chosen in order to be able to observe the formation of the spheroids in the containers (3).

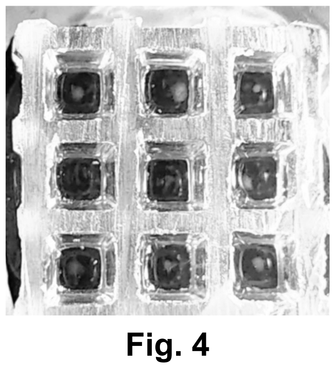

Figure 4 shows a photograph of the formation of various spheroids of PANC-1 pancreatic cancer cells, induced ultrasonically in a period of time of approximately 4 minutes, demonstrating the correct functioning of the device of the invention.

## Claims

1. An ultrasonic device for generating three-dimensional cell spheroids from microelements suspended in liquids, **characterised in that** it comprises:

   - a polymer structure (2) comprising a set of containers (3), wherein each container (3) comprises a base and sides and is intended to contain a liquid, the difference between the acoustic impedance of the polymer structure (2) and the liquid being less than 60%,
   - an ultrasonic actuator (1) concentrically coupled to the containers (3) at their base to transfer mechanical vibrations thereto, and
   - a signal generator (4), connected to the ultrasonic actuator (1), configured to provide sinusoidal electrical waves at a frequency between 450 kHz and 520 kHz that maximises the conductance of the ultrasonic actuator (1).

2. The device according to claim 1, wherein the ultrasonic actuator (1) comprises a piezoelectric ceramic, which is resonant in a thickness mode.

3. The device according to claim 1, wherein the frequency of the electrical sinusoidal waves generated by the signal generator (4) is selected from 469kHz, 491kHz and 508kHz.

4. The device according to claim 1, wherein each container (3) of the polymeric structure (2) comprises a base with a square cross section, with sides between 2 and 4 mm long, and four sides which are flat walls between 8 and 12 mm high.

5. The device according to claim 1, wherein the polymer structure (2) comprises a single container (3).

6. The device according to claim 1, wherein the polymer structure (2) comprises four containers (3) arranged in a 2x2 matrix.

7. The device according to claim 1, wherein the polymer structure (2) comprises nine containers (3) arranged in a 3x3 matrix.

8. The device according to claim 1, wherein the polymer structure (2) is made of polyethylene with a density $\rho$ = 1.01 g/cm.

9. Method for generating three-dimensional cell spheroids from microelements suspended in liquids, which uses the device according to any of the preceding claims, and comprising the steps of:

   - applying sinusoidal electrical waves to the

**EP 4 745 234 A1**

containers (3) of the polymer structure (2) with the ultrasonic actuator (1) and generated in the signal generator with a variable frequency between 450 kHz and 520 kHz,
- obtaining a measurement of the conductance (G) of the ultrasonic actuator (1) for each of the applied frequencies,
- determining at least one operating frequency for which the conductance is maximal, and
- applying the sinusoidal electrical wave at the operating frequency to the containers (3) to obtain the spheroids.

**FIG. 1**

**FIG. 2**

**FIG. 3**

**Fig. 4**

# INTERNATIONAL SEARCH REPORT

| International application No. |
|---|
| PCT/ES2024/070450 |

## A. CLASSIFICATION OF SUBJECT MATTER

*C12N13/00* (2006.01)
*C12M1/42* (2006.01)
According to International Patent Classification (IPC) or to both national classification and IPC

## B. FIELDS SEARCHED

Minimum documentation searched (classification system followed by classification symbols)
C12N, C12M

Documentation searched other than minimum documentation to the extent that such documents are included in the fields searched

Electronic data base consulted during the international search (name of data base and, where practicable, search terms used)

EPODOC, INVENES

## C. DOCUMENTS CONSIDERED TO BE RELEVANT

| Category* | Citation of document, with indication, where appropriate, of the relevant passages | Relevant to claim No. |
|---|---|---|
| A | US 2014038257 A1 (SUBRAMANIAN ET AL.) 06/02/2014, page 2, paragraph [27] - page 4, paragraph[46]; page 5, paragraph 56; figures 1A, 1B. | 1-9 |
| A | US 2003153077 A1 (PITT ET AL.) 14/08/2003, page 2, paragraph [16] - page 3, paragraph [29]; page 6, paragraphs [66 - 73]. | 1-9 |
| A | OLOFSSON, KARL et al.: "Ultrasound-based scaffold-free core-shell multicellular tumor spheroid formation". Micromachines, 2021, Vol. 12, N° 3, page 329. | 1-9 |
| A | SHAO, CHANGMIN et al.: "Development of cell spheroids by advanced technologies". Advanced Materials Technologies, 2020, Vol. 5, N° 9, page 2000183. | 1-9 |

☒ Further documents are listed in the continuation of Box C.          ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 04/10/2024 | **(08/10/2024)** |

| Name and mailing address of the ISA/ | Authorized officer |
|---|---|
| OFICINA ESPAÑOLA DE PATENTES Y MARCAS Paseo de la Castellana, 75 - 28071 Madrid (España) Facsimile No.: 91 349 53 04 | R. San Vicente Domingo Telephone No. 913498525 |

Form PCT/ISA/210 (second sheet) (July 2022)

## INTERNATIONAL SEARCH REPORT

| | International application No. |
|---|---|
| | PCT/ES2024/070450 |

C (continuation).          DOCUMENTS CONSIDERED TO BE RELEVANT

| Category * | Citation of documents, with indication, where appropriate, of the relevant passages | Relevant to claim No. |
|---|---|---|
| A | CHEN, KEJIE et al.: "Rapid formation of size-controllable multicellular spheroids via 3D acoustic tweezers". Lab On A Chip, 2016, Vol. 16, N° 14, pages 2636-2643. | 1-9 |
| A | US 2018251718 A1 (LABRUZZO) 06/09/2018, Page 2, paragraph [32] - page 3, paragraph [47]; figure 1. | 1-9 |

Form PCT/ISA/210 (continuation of second sheet) (July 2022)

## INTERNATIONAL SEARCH REPORT

Information on patent family members

| | | International application No. |
|---|---|---|
| | | PCT/ES2024/070450 |

| Patent document cited in the search report | Publication date | Patent family member(s) | Publication date |
|---|---|---|---|
| US2014038257 A1 | 06.02.2014 | NONE | |
| US2003153077 A1 | 14.08.2003 | WO03089581 A2 | 30.10.2003 |
| | | WO03089581 A3 | 26.02.2004 |
| | | AU2003223608 A1 | 03.11.2003 |
| | | AU2003223608 A8 | 03.11.2003 |
| US2018251718 A1 | 06.09.2018 | HK1250048 A1 | 23.11.2018 |
| | | CN107624128 A | 23.01.2018 |
| | | EA201792053 A1 | 31.01.2018 |
| | | EA033774 B1 | 25.11.2019 |
| | | EP3271450 A1 | 24.01.2018 |
| | | EP3271450 B1 | 06.11.2019 |
| | | WO2016147156 A1 | 22.09.2016 |

Form PCT/ISA/210 (patent family annex) (July 2022)